# FASCICULE DE BREVET EUROPEEN

(11) **EP 0 692 484 B1**
(45) Date de publication et mention de la délivrance du brevet: **31.03.1999**
(21) Numéro de dépôt: 95401666.3
(22) Date de dépôt: 12.07.1995
(51) Int. Cl.: C07D 513/04, A61K 31/54

(54) **Nouveau dérivé de benzothiadiazine, son procédé de préparation et les compositions pharmaceutiques qui le contiennent**
Benzothiadiazinderivate, Verfahren zu ihrer Herstellung und diese enthaltende pharmazeutische Zuzammensetzungen
Benzothiadiazine derivative process for its preparation and pharmaceutical compositions containing it

(30) Priorité: 12.07.1994 FR 9408603
(43) Date de publication de la demande: 17.01.1996
(73) Titulaire: ADIR ET COMPAGNIE, 92415 Courbevoie Cédex (FR)
(72) Inventeur: Cordi, Alex, F-92150 Suresnes (FR); Spedding, Michael, F-78110 Le Vesinet (FR); Serkiz, Bernard, F-77170 Servon Brie Comte Robert (FR); Lepagnol, Jean, F-28210 Chaudon (FR); Desos, Patrice, F-92400 Courbevoie (FR); Morain, Philippe, F-92130 Issy les Moulineaux (FR)

(56) Documents cités:
- CHEMICAL ABSTRACTS, vol. 90, no. 5, 29 Janvier 1979, Columbus, Ohio, US; abstract no. 33765, R. CAMERONI ET AL 'Heteropolycyclic systems. VI. 4-Propyl-2,3,3a,4,tetrahydro- 1H-pyrrolo(2,1-c)(1,2,4)benzothiadiazine 5,5-dioxide and its derivatives. Cardiovascular effects of 4-alkyltetrahydropyrrolobenzothiadiazines on anesthetized rats.' page 15 ; & FARMACO, EDIZIONE SCIENTIFICA, vol.33, no.9, 1978, PAVIA IT pages 713 - 720
- CHIRALITY, vol.3, no.1, 1991 pages 2 - 13 E. NUSSER ET AL 'Excavations in drug chirality: 1. Cyclothiazide'

## Description

La présente invention concerne un nouveau dérivé de benzothiadiazine, son procédé de préparation et les compositions pharmaceutiques qui le contiennent.

Il est désormais reconnu que les aminoacides excitateurs et tout particulièrement le glutamate, jouent un rôle crucial dans les processus physiologiques de plasticité neuronale et dans les mécanismes sous-tendant l'apprentissage et la mémoire. Récemment, les études pathophysiologiques ont indiqué clairement qu'un déficit de la neurotransmission glutamatergique est associé étroitement avec le développement de la maladie d'Alzheimer (Neuroscience and Biobehavioral reviews, 1992, 16, 13-24 ; Progress in Neurobiology, 1992, 39, 517-545).

Par ailleurs, d'innombrables travaux ont démontré durant les dernières années l'existence de sous-types réceptoriels aux aminoacides excitateurs et de leurs interactions fonctionnelles (Molecular Neuropharmacology, 1992, 2, 15-31).

Parmi ces récepteurs, le récepteur à l'AMPA ("α-amino-3-hydroxy-5-methyl-4-isoxazolepropionic acid") apparait être le plus impliqué dans les phénomènes d'excitabilité neuronale physiologique et notamment dans ceux impliqués dans les processus de mémorisation. Pour exemple, l'apprentissage a été montré comme étant associé à l'augmentation de la liaison de l'AMPA à son récepteur dans l'hippocampe, l'une des zones cérébrales essentielles aux processus mnémocognitifs. De même, les agents nootropes tels que l'aniracetam ont été décrits très récemment comme modulant positivement les récepteurs AMPA des cellules neuronales (Journal of Neurochemistry, 1992, 58, 1199-1204).

Très récemment, des composés de structure benzamide ont été décrits pour posséder ce même mécanisme d'action et pour améliorer les performances mnésiques (Synapse, 1993, 15, 326-329). Le composé BA 74, en particulier, est le plus actif parmi ces nouveaux agents pharmacologiques.

Plus spécifiquement, la présente invention concerne l'isomère S du 5,5-dioxo-2,3,3a,4-tétrahydro-1H-pyrrolo[2,1-c][1,2,4]benzothiadiazine de formule (I) : 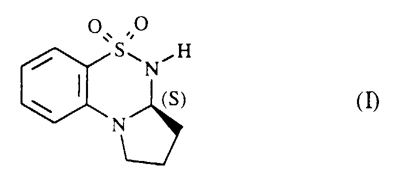 ainsi que ses sels d'addition à un acide ou à une base pharmaceutiquement acceptable.

Ce composé, sous forme racémique, a été décrit dans la littérature par M.T. BERNABEI et coll. (II Farmaco, Ed. Sc., 1976, 31, fasc. 7, 508-516).
Ce composé, sous forme racémique, est connu, de plus, pour avoir des activités hypotensives et bradycardisantes comme l'ont montré R. CAMERONI et coll. (II Farmaco, Ed. Sc., 1978, vol.33, fasc. 9, 713-720).
Or, la demanderesse a présentement découvert que l'isomère S de ce composé était non seulement nouveau mais possédait de façon surprenante une puissante activité facilitatrice sur le courant AMPA qui le rend utile dans le traitement des maladies impliquant le récepteur à l'AMPA.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

La présente invention concerne également le procédé d'obtention de cet isomère caractérisé en ce que l'on met en réaction le composé de formule (II) : 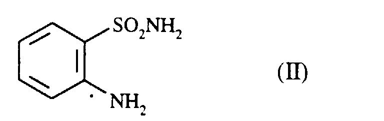 avec le chlorure d'acide de formule (III) en présence de triéthylamine, en milieu tétrahydrofurane :

Cl-(CH₂)₃-COCl (III)

pour conduire au composé de formule (IV) : 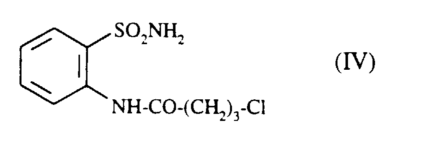 qui est alors cyclisé en milieu basique, pour conduire au composé de formule (V) : 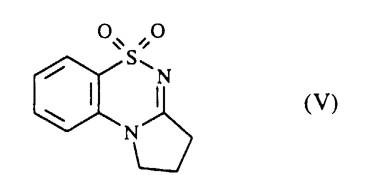 composé de formule (V) qui subit :
- soit une réduction, en milieu alcoolique, en présence de borohydrure de sodium, pour conduire au composé de formule (VI), sous forme racémique, 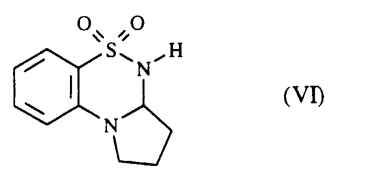 dont on sépare les isomères par chromatographie liquide sur phase chirale, pour conduire à l'isomère S de formule (I), énantiomériquement pur,
- soit une réduction stéréospécifique en présence d'hydrogène utilisant un catalyseur métallique dérivé de ruthénium, de rhodium, de palladium, de platine ou d'irridium compléxé à un ligand chiral comme 2,2'-bis(diphénylphosphino)-1,1'-binaphtyl ou BINAP, ou, en présence d'hydrure de bore ou d'aluminium ayant réagi préalablement avec un ligand chiral comme le 4-diméthylamino-1,2-diphényl-3-méthyl-2-butanol, conduisant directement à l'isomère S de formule (I), énantiomériquement pur,
composé de formule (I), que l'on purifie, si on le souhaite, par une technique classique de purification, et que l'on transforme, le cas échéant, en sel d'acide ou de base pharmaceutiquement acceptable.

Le composé de formule (I) peut également être obtenu par synthèse stéréospécifique mettant en jeu un auxiliaire chiral comme l'α-méthylbenzylamine que l'on met en réaction avec le chlorure de l'acide 2-aminobenzènesulfonique. L'aminosulfonamide formé est mis en réaction avec le chlorure de l'acide 4-chlorobutanoïque, réduit, cyclisé et épimérisé par traitement successif avec un agent de réduction comme le borohydrure de sodium, puis avec une base comme l'hydroxyde de sodium. Le diastéréoisomère formé est alors éventuellement purifié et l'auxiliaire chiral est éliminé par hydrogénolyse en présence d'un catalyseur métallique.

Le composé de la présente invention présente des propriétés pharmacologiques intéressantes puisqu'il peut potentialiser sélectivement les phénomènes électrophysiologiques excitateurs induits par l'AMPA, soit dans l'oocyte de Xenopus exprimant les récepteurs au glutamate par injection d'ARNm de cortex de Rat, soit dans l'hippocampe lors de la stimulation électrique des voies de neurotransmission glutamatergique.

Comme le montrent les études électrophysiologiques conduites vis-à-vis de l'excitabilité induite par l'AMPA exprimé dans l'oocyte de Xenopus ou présent naturellement dans l'hippocampe, ces effets sont toujours supérieurs à ceux des composés pris en référence et confèrent au composé de l'invention des potentialités pharmacologiques et thérapeutiques vis-à-vis des troubles induits par le dysfonctionnement de la neurotransmission glutamatergique et notamment liés au récepteur AMPA.

La neurotransmission glutamatergique est désormais largement démontrée comme cruciale pour les processus physiologiques de l'apprentissage et de la mémoire, et plus largement pour les processus gouvernant les facultés d'attention, de concentration et de vigilance. Plus particulièrement, le sous-type réceptoriel dénommé AMPA semble jouer un rôle fondamental pour ces processus. Le composé de la présente invention a montré des effets pharmacologiques intéressants puisqu'il peut sélectivement faciliter l'activation de ce récepteur AMPA. Ces effets sont, de façon surprenante, beaucoup plus intenses que ceux des composés de référence anciennement décrits (Diazoxide et Aniracetam) ou récemment proposés (BA 74), ou bien ceux observés avec le mélange racémique.

Le composé de l'invention est donc utile en tant qu'agent facilitateur de l'activation induite par l'acide glutamique au niveau des récepteurs AMPA et constitue donc un agent thérapeutique pour le traitement et la prévention des pathologies liées au dysfonctionnement de la neurotransmission glutamatergique telles que :
- les désordres mnémocognitifs associés à l'âge et aux syndromes anxieux ou dépressifs,
- les déficits mnésiques des maladies neurodégénératives progressives comme par exemple la maladie d'Alzheimer, la maladie de Pick, la chorée d'Huntington et la schizophrénie,
- les séquelles des maladies neurodégénératives algües comme par exemple l'ischémie et l'épilepsie.

La présente invention a également pour objet les compositions pharmaceutiques contenant le composé de formule (I), en combinaison avec un ou plusieurs excipients ou véhicules pharmaceutiquement acceptables.

Parmi les compositions pharmaceutiques selon l'invention, on pourra citer, à titre d'exemples et de façon non limitative, celles qui conviennent à une administration par voie orale, rectale, nasale ou parentérale et notamment les comprimés, les dragées, les gélules, les paquets, les sachets, les granules, les pilules, les granulés, les suppositoires, les aérosols, les capsules et les solutions injectables ou buvables.

La posologie varie d'un individu à l'autre, selon l'âge, le poids et le sexe du malade, la voie d'administration choisie, la nature et l'intensité de l'affection. Les doses utilisées s'échelonnent entre 1 et 500 mg pour un traitement, divisibles en 1 à 3 prises par 24 h.

Les exemples suivants illustrent l'invention et ne la limitent en aucune façon.

### Exemple 1: (3aS)-5,5-Dioxo-2,3,3a,4-tétrahydro-1H-pyrrolo[2,1-c][1,2,4]benzothiadiazine

### Stade A : 5,5-Dioxo-2,3-dihydro-1H-pyrrolo[2,1-c][1,2,4]benzothiadiazine

5,8 mmoles de 2-aminobenzènesulfonamide sont dissoutes dans du tétrahydrofurane (THF) à température ambiante. Cette solution est refroidie au bain de glace et successivement sont ajoutées 6 mmoles de chlorure de l'acide 4-chlorobutanoïque et 6 mmoles de triéthylamine. Le mélange est agité 16 heures à température ambiante, filtré et évaporé. Le solide jaune obtenu est repris dans 40 ml de soude 1N et agité à température ambiante. Après dissolution complète, un précipité blanc apparait ; il est filtré et conduit au produit attendu après cristallisation dans un mélange éthanol/eau (4/1).

### Stade B : 5,5-Dioxo-2,3,3a,4-tétrahydro-1H-pyrrolo[2,1-c][1,2,4]benzothiadiazine

Le composé obtenu au stade précédent est dissous dans 30 ml d'éthanol. La solution est additionnée de 26 mmoles d'hydrure de sodium, agitée 90 minutes et évaporée. Le résidu ainsi obtenu est repris par de l'eau, extrait au dichlorométhane. Après séchage et évaporation des phases organiques, on obtient le produit attendu qui est cristallisé dans l'acétate d'éthyle.
*Point de fusion : 202°C*

| *Microanalyse élémentaire :* | | | | |
|---|---|---|---|---|
| | *C* % | *H* % | *N* % | *S* % |
| *calculé* | *53,55* | *5,39* | *12,49* | *14,30* |
| *trouvé* | *53,98* | *5,40* | *12,38* | *14,23* |

### Stade C : (3aS)-5,5-Dioxo-2,3,3a,4-tétrahydro-1H-pyrrolo[2,1-c][1,2,4]benzothiadiazine

Les énantiomères du composé obtenu au stade précédent sont séparés par chromatographie liquide chirale préparative sur colonne Chiralpak AS (50 x 2 cm). L'élution est réalisée en utilisant comme solvant un mélange heptane/éthanol (1/2), à la vitesse de 1,5 ml/min. La séparation est réalisée avec des échantillons contenant 80 mg du mélange racémique dans 2,5 ml d'acétone. Les fractions correspondant à chaque pic d'élution sont rassemblées et évaporées. L'isomère R est élué le premier, l'isomère actif S est élué en second. La pureté énantiomérique de chaque isomère est alors vérifiée par chromatographie HPLC chirale, analytique dans les mêmes conditions que celles utilisées pour la séparation préparative. Les énantiomères obtenus sont recristallisés dans de l'acétate d'éthyle. La configuration absolue de l'énantiomère actif (S) a été déterminée sur un monocristal du produit par diffraction de Rayons X.

### Isomère S :

*Point de fusion : 218°C*

| *Microanalyse élémentaire :* | | | | |
|---|---|---|---|---|
| | *C* % | *H* % | *N* % | *S* % |
| *calculé* | *53,55* | *5,40* | *12,49* | *14,30* |
| *trouvé* | *53,80* | *5,44* | *12,02* | *14,05* |

### Isomère R :

*Point de fusion : 219°C*

| *Microanalyse élémentaire :* | | | | |
|---|---|---|---|---|
| | *C* % | *H* % | *N* % | *S* % |
| *calculé* | *53,55* | *5,40* | *12,49* | *14,30* |
| *trouvé* | *53,80* | *5,57* | *12,63* | *14,26* |

### Etude pharmacologique du composé de l'invention

### Exemple 2 : Etude des courants excitateurs induit par l'AMPA dans les oocytes de Xenopus

### a - Méthode :

Des ARNm sont préparés à partir de cortex cérébral de rat mâle Wistar par la méthode au guanidium thiocyanate/phénol/chloroforme. Les ARNm Poly (A⁺) sont isolés par chromatographie sur oligo-dT cellulose et injectés à raison de 50 ng par oocyte. Les oocytes sont laissés 2 à 3 jours en incubation à 18°C pour permettre l'expression des récepteurs puis stockés à 8-10°C.

L'enregistrement électrophysiologique est réalisé dans une chambre en plexiglass® à 20-24°C en milieu OR2 (J. Exp. Zool., 1973, 184, 321-334) par la méthode du "voltage-clamp" à 2 électrodes, une 3ème électrode placée dans le bain servant de référence.

Tous les composés sont appliqués via le milieu d'incubation et le courant électrique est mesuré à la fin de la période d'application. L'AMPA est utilisé à la concentration de 30 µM. Pour chaque composé étudié, on définit la concentration doublant (EC2X) ou quintuplant (EC5X) l'intensité du courant induit par l'AMPA seul (50 à 100 nA).

### b - Résultats :

Le composé de l'invention potentialise très fortement les effets excitateurs de l'AMPA et son activité est très nettement supérieure à celles des composés de référence ainsi qu'à celles du racémique ou de l'isomère R comme indiqué dans le tableau suivant :

| Composé | EC2X (µM) | EC5X (µM) |
|---|---|---|
| Exemple 1 (isomère S) | 35 | 70 |
| Mélange racémique | 60 | 160 |
| Isomère R | 300 | >1000 |
| BA 74 | 45 | 110 |
| Diazoxide | 400 | 1000 |
| Aniracétam | 1300 | 5000 |

De plus, cette activité facilitatrice du composé de l'invention est sélective pour le récepteur AMPA car aucune potentialisation du courant n'est observée quand l'agoniste glutamatergique utilisé est le NMDA ou le Kainate (KA).

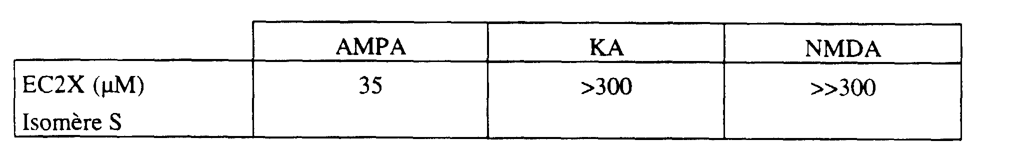

### Exemple 3 : Etude des potentiels excitateurs synaptiques induits par la stimulation électrique sur coupe d'hippocampe

### a - Méthode :

Des coupes transversales d'hippocampe (500 µM) de rat mâle Wistar sont préparées à l'aide d'un "tissu chopper" puis incubées durant 45 minutes dans un milieu sans calcium contenant 10 mM Mg⁺⁺. Elles sont ensuite stabilisées dans du Krebs ajusté à pH 7,35 et oxygéné par O₂/CO₂ (95 % / 5 %) à température ambiante.

Les coupes sont mises en submersion à 35°C et les potentiels excitateurs post-synaptiques (PEPS) sont enregistrés dans le champ dendritique des cellules granulaires du dentate gyrus lors de stimulation (50-100 µA, 50 µsec) toutes les 30 secondes de la voie perforante par électrode bipolaire de tungstène.

L'acquisition et l'analyse des PEPS sont réalisées grâce à un convertisseur A-D, une interface TL-1 et un software "pCLAMP".

L'amplitude et la durée des PEPS sont évaluées sur l'onde négative par rapport au courant de base.

Les composés sont appliqués durant 10 à 20 minutes dans le bain de superfusion contenant du MgSO₄ (1 mM) afin de bloquer l'activation des récepteurs NMDA. Pour chaque composé, on définit la concentration augmentant de 50 % l'amplitude (A50) du PEPS.

### b - Résultats :

Le composé de l'invention augmente l'amplitude du PEPS à doses plus faibles que les composés de référence, le racémique ou l'isomère R comme le montre le tableau suivant :

| Composé | A50 (µM) |
|---|---|
| Exemple 1 (Isomère S) | 90 |
| Racémique | >300 |
| Isomère R | >>1000 |
| BA 74 | >300 |
| Diazoxide | 560 |
| Aniracétam | 3000 |

## Revendications

1. Composé de formule (I) : 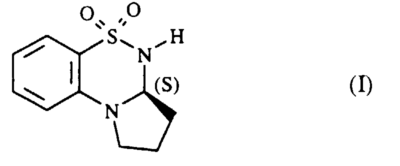 sous la forme de son énantiomère S, ainsi que ses sels d'addition à un acide ou à une base pharmaceutiquement acceptable.

2. Procédé de préparation du composé de formule (I) caractérisé en ce que l'on met en réaction le composé de formule (II) : 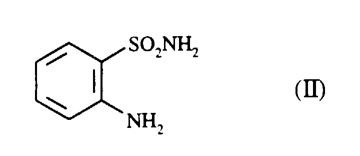 avec le chlorure d'acide de formule (III) en présence de triéthylamine, en milieu tétrahydrofurane :
Cl-(CH₂)₃-COCl (III)
pour conduire au composé de formule (IV) : 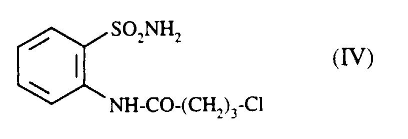 qui est alors cyclisé en milieu basique, pour conduire au composé de formule (V) : 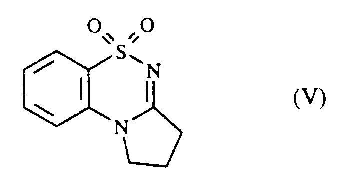 composé de formule (V) qui subit :
- soit une réduction, en milieu alcoolique, en présence de borohydrure de sodium,
pour conduire au composé de formule (VI), sous forme racémique, 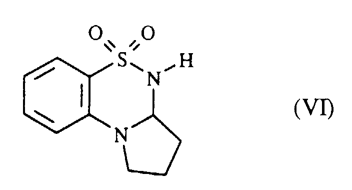 dont on sépare les isomères par chromatographie liquide sur phase chirale,
pour conduire à l'isomère S de formule (I), énantiomériquement pur,
- soit une réduction stéréospécifique en présence d'hydrogène utilisant un catalyseur métallique dérivé de ruthénium, de rhodium, de palladium, de platine ou d'irridium compléxé à un ligand chiral comme 2,2'-bis(diphénylphosphino)-1,1'-binaphtyl ou BINAP, ou, en présence d'hydrure de bore ou d'aluminium ayant réagi préalablement avec un ligand chiral comme le 4-diméthylamino-1,2-diphényl-3-méthyl-2-butanol, conduisant directement à l'isomère S de formule (I) énantiomériquement pur,
composé de formule (I), que l'on purifie, si on le souhaite, par une technique classique de purification, et que l'on transforme le cas échéant en sel d'acide ou de base pharmaceutiquement acceptable.

3. Composition pharmaceutique contenant le composé de formule (I) selon la revendication 1, en combinaison avec un ou plusieurs excipients ou véhicules pharmaceutiquement acceptables.

4. Composition pharmaceutique selon la revendication 3 contenant le composé de formule (I) selon la revendication 1 utile pour le traitement et la prévention des pathologies liées au dysfonctionnement de la neurotransmission glutamatergique.

5. Composition pharmaceutique selon la revendication 4 utile pour le traitement et la prévention des désordres mnémocognitifs associés à l'âge, aux syndromes anxieux ou dépressifs, aux maladies neurodégénératives progressives, à la maladie d'Alzheimer, à la maladie de Pick, à la chorée d'Huntington, à la schizophrénie, aux séquelles des maladies neurodégénératives aigües, aux séquelles de l'ischémie et aux séquelles de l'épilepsie.

## Patentansprüche

1. Verbindung der Formel (I): 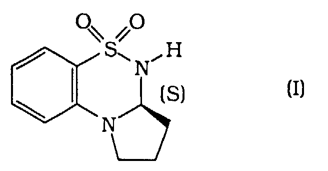 in Form des S-Isomeren, sowie deren Additionssalze mit einer pharmazeutisch annehbmaren Säure oder Base.

2. Verfahren zur Herstellung der Verbindung der Formel (I), dadurch gekennzeichnet, daß man die Verbindung der Formel (II): 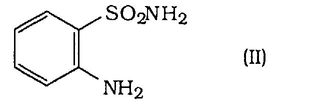 mit dem Säurechlorid der Formel (III):
Cl - (CH₂)₃ - COCl (III)
in Gegenwart von Triethylamin in Tetrahydrofuran-Medium umsetzt zur Bildung der Verbindung der Formel (IV): 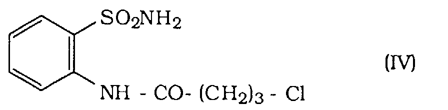 welche anschließend in basischem Medium cyclisiert wird zur Bildung der Verbindung der Formel (V): 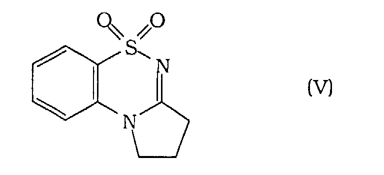 welche Verbindung der Formel (V):
- entweder einer Reduktion in alkoholischem Medium in Gegenwart von Natriumborhydrid unterworfen wird zur Bildung der Verbindung der Formel (VI) in Form des Racemats 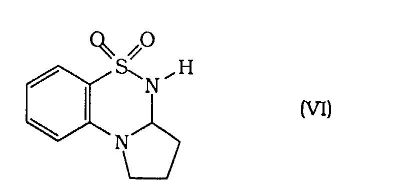 welche man durch Flüssigchromatographie auf chiraler Phase in die Isomeren auftrennt zur Bildung des enantiomerenreinen Isomeren S der Formel (I),
- oder einer stereospezifischen Reduktion in Gegenwart von Wasserstoff unter Verwendung eines Metallkatalysators, der von Ruthenium, Rhodium, Palladium, Platin oder Iridium abgeleitet und mit einem chiralen Liganden, wie 2,2'-Bis(diphenylphosphino)-1,1'-binaphthyl oder BINAP komplexiert worden ist, oder in Gegenwart von Borhydrid oder Aluminiumhydrid, welches zuvor mit einem chiralen Liganden, wie 4-Dimethylamino-1,2 -diphenyl-3-methyl-2 -butanol umgesetzt worden ist, einer stereospezifischen Reduktion unterworfen wird, welche direkt zu dem enantiomerenreinen Isomeren S der Formel (I) führt,
welche Verbindung der Formel (I) man gewünschtenfalls mit Hilfe einer klassischen Reinigungsmethode reinigt und gegebenenfalls in das Salz mit einer pharmazeutisch annehmbaren Säure oder Base umwandelt.

3. Pharmazeutische Zubereitung enthaltend die Verbindung der Formel (I) nach Anspruch 1 in Kombination mit einem oder mehreren pharmazeutisch annehmbaren Trägermaterialien oder Bindemitteln.

4. Pharmazeutische Zubereitung nach Anspruch 3, enthaltend die Verbindung der Formel (I) nach Anspruch 1 zur Behandlung und zur Vorbeugung von pathologischen Zuständen, die mit einer Dysfunktion der glutamatergischen Neurotransmission verknüpft sind.

5. Pharmazeutische Zubereitung nach Anspruch 4 zur Behandlung und zur Vorbeugung von Erkenntnis/Gedächtnis-Störungen, die mit dem Alter verbunden sind, Angstsyndromen oder Depressionszuständen, progressiven neurodegenerativen Erkrankungen, der Alzheimerschen Krankheit, der Pickschen Krankheit, der Huntington-Chorea, der Schizophrenie, von Folge erscheinungen von akuten neurodegenerativen Erkrankungen, von Folgeerscheinungen der Ischämie und von Folgeerscheinungen der Epilepsie.

## Claims

1. Compound of formula (I): 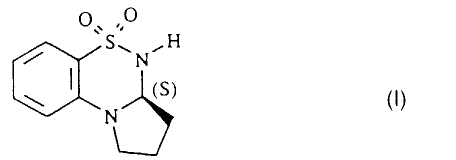 in the form of its S enantiomer, and its addition salts with a pharmaceutically acceptable acid or base.

2. Process for the preparation of the compound of formula (I), characterised in that the compound of formula (II): 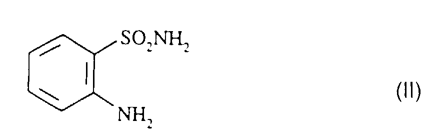 is reacted with the acid chloride of formula (III) in the presence of triethylamine, in a tetrahydrofuran medium:
Cl - (OH₂)₃ - COCl (III)
to yield the compound of formula (IV): 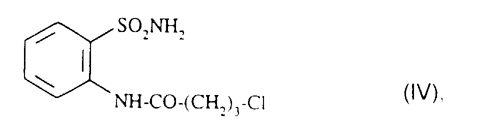 which is then cyclised in a basic medium to yield the compound of formula (V): 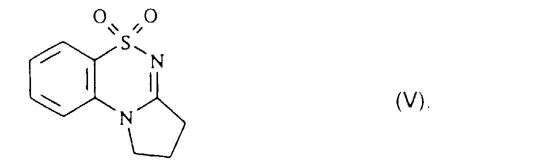 which compound of formula (V) is subjected:
- either to reduction, in an alcoholic medium, in the presence of sodium borohydride, to yield the compound of formula (VI) in racemic form 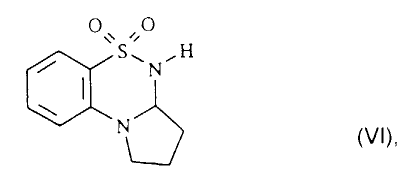 which is separated into its isomers by liquid chromatography on a chiral phase to yield the enantiomerically pure S isomer of formula (I),
- or to stereospecific reduction in the presence of hydrogen using a metal catalyst derived from ruthenium, rhodium, palladium, platinum or iridium complexed to a chiral ligand, such as 2,2'-bis(diphenylphosphino)-1,1'-binaphthyl or BINAP, or in the presence of boron hydride or aluminium hydride which has previously been reacted with a chiral ligand, such as 4-dimethylamino-1,2-diphenyl-3-methyl-2-butanol, yielding the enantiomerically pure S isomer of formula (I) directly,
which compound of formula (I) is purified, if desired, by a customary purification technique and is converted, where appropriate, into a salt with a pharmaceutically acceptable acid or base.

3. Pharmaceutical composition comprising the compound of formula (I) according to claim 1, in combination with one or more pharmaceutically acceptable excipients or carriers.

4. Pharmaceutical composition according to claim 3 comprising the compound of formula (I) according to claim 1, for use in the treatment and prevention of pathologies associated with dysfunctioning of glutamatergic neurotransmission

5. Pharmaceutical composition according to claim 4 for use in the treatment and prevention of mnemocognitive disorders associated with age, anxiety or depressive syndromes, progressive neurodegenerative diseases, Alzheimer's disease, Pick's disease, Huntington's chorea, schizophrenia, the after-effects of acute neurodegenerative diseases, the after-effects of ischaemia and the after-effects of epilepsy.
